# EUROPÄISCHE PATENTSCHRIFT

(11) **EP 3 911 623 B1**
(45) Veröffentlichungstag und Bekanntmachung des Hinweises auf die Patenterteilung: **19.03.2025**
(21) Anmeldenummer: 19701476.4
(22) Anmeldetag: 17.01.2019
(51) Int. Cl.: C07C 45/34, C07C 49/607

(54) **VERFAHREN ZUR HERSTELLUNG UNGESÄTTIGTER MAKROZYKLISCHER KETONE (II)**
METHOD FOR PREPARING UNSATURATED MACROCYCLIC KETONES (II)
PROCÉDÉ POUR PRODUIRE DES CÉTONES (II) MACROCYCLIQUES INSATURÉES

(43) Veröffentlichungstag der Anmeldung: 24.11.2021
(73) Patentinhaber: Symrise AG, 37603 Holzminden Niedersachsen (DE)
(72) Erfinder: BRUNZEL, Tom, 29486 Summerville (US); KÖCKRITZ, Angela, 12437 Berlin (DE)
(74) Vertreter: Fabry, Bernd
(86) Internationale Anmeldenummer: PCT/EP2019/051091
(87) Internationale Veröffentlichungsnummer: WO 2020/147951

(56) Entgegenhaltungen:
- EP-A1- 2 364 965
- JP-A- H 069 481
- MOOKHERJEE B D ET AL: "Synthesis of racemic muscone and cyclopentadecanone (exaltone) from 1,9-cyclohexadecadiene", JOURNAL OF ORGANIC CHEMISTRY, AMERICAN CHEMICAL SOCIETY, US, vol. 36, no. 22, 1 January 1971 (1971-01-01), pages 3266 - 3270, XP002432756, ISSN: 0022-3263, DOI: 10.1021/JO00821A003

## Beschreibung

### GEBIET DER ERFINDUNG

Die Erfindung befindet sich auf dem Gebiet der Herstellung von ungesättigten makrozyklischen Monoketonen aus makrozyklischen Dienen in einer einstufigen Oxidation in Gegenwart eines Oxidationsmittels, eines Palladium(II)-Salzes und/oder eines Palladium(II)-Komplexes, eines Co-Katalysators und eines Lösungsmittels.

### TECHNOLOGISCHER HINTERGRUND

Ketone sind als Lösungsmittel und chemische Rohstoffe geeignet und werden daher in verschiedenen Bereichen eingesetzt. Solche Ketone werden im Allgemeinen durch zweistufige Reaktionsverfahren hergestellt, bei denen ein durch Hydratisieren eines Olefins erzeugter Alkohol dehydriert wird. Mittlerweile sind als einfachere Verfahren auch einstufige Reaktionsverfahren bekannt, bei denen ein Olefin direkt oxidiert wird.

Zweistufige Verfahren zur Herstellung von makrozyklischen Monoketonen sind aus dem Stand der Technik bekannt. Die technische Synthese von makrozyklischen Ketonen gelingt beispielsweise über eine selektive Monoepoxidierung und Umlagerung des Epoxids zum Keton in Gegenwart von Alkali- oder Erdalkalihalogeniden, wie in B. D. Mookherjee, R. W. Trenkle, R. R. Patel, J. Org. Chem. 36 (1971), 3266 beschrieben. Eine Reduktion der Epoxygruppe zu einer Hydroxygruppe, gefolgt von der anschließenden Oxidation zum Keton wird beispielsweise in US 3,718,696 beschrieben.

Das Wacker-Verfahren, unter Verwendung eines PdCl₂ / CuCl₂-Katalysators, ist als eines der Verfahren zur direkten Oxidation eines Olefins bekannt. Dieses Verfahren ist wirksam für die Oxidation von terminalen Olefinen mit jeweils einer Kohlenstoff-Kohlenstoff-Doppelbindung (nachfolgend als "C = C-Bindung" abgekürzt) an einem Ende eines Moleküls. Jedoch erzielt dieses Verfahren nur eine geringe Reaktivität, wenn es für die Oxidation von internen Olefinen verwendet wird, die jeweils eine C = C-Bindung an einer anderen Position als ihren Enden aufweisen. Dieses Verfahren erzielt ebenso bei einer Erhöhung der Anzahl der Kohlenstoffatome des Ausgangsmaterials keine zufriedenstellenden Ergebnisse, da die Reaktionsgeschwindigkeit deutlich gesenkt wird. Aus diesem Grund ist auf dem industriellen Gebiet die Verwendung des Wacker-Verfahrens nur auf die Herstellung von niederen Carbonylverbindungen wie Acetaldehyd und Aceton beschränkt, die durch Oxidation von niederen terminalen Olefinen erhalten werden.

### RELEVANTER STAND DER TECHNIK

Kaneda *et al.* entwickelte eine Methode, welche kein Cooxidans für die Reoxidation von Pd(0) benötigt, sondern in welcher Sauerstoff Pd(0) zu Pd(II) direkt reoxidiert. Hierbei wird ein Dimethylacetamid/H₂O-Gemisch bei 80 °C verwendet (T. Mitsudome, T. Umetani, N. Nosaka, K. Mori, T. Mizugaki, K. Ebitani, K. Kaneda, Angew. Chem. Int. Ed. 45 (2006) 481*).* Dieses Verfahren gestattet auch die Oxidation linearer interner Olefine und cyclischer Olefine, wie beispielsweise Cyclohexen *(*T. Mitsudome, K. Mizumoto, T. Mizugaki, K. Jitsukawa, K. Kaneda, Angew. Chem. Int. Ed. 49 (2010) 1238*).* Erfolgreich waren hierbei jedoch nur chlorhaltige Palladium-Katalysatoren. Eigene Untersuchungen zeigten, dass aus makrozyklischen Olefinen und Dienen als Substrat nach dieser Methode, auch bei Verwendung eines Cooxidationsmittels, nur unzureichende Ausbeuten an den gewünschten Ketonen erzielt werden.

Eine weitere Variation der Wacker-Oxidation von Olefinen unter Verwendung kationischer Palladiumkomplexe wird in der US 20140194604 beschrieben. Hierbei werden jedoch keine makrozyklischen Diene als Ausgangssubstrat verwendet.

Ebenso beschreibt die WO 2010 061807 eine Abwandlung des Wacker-Verfahrens in welcher auch makrozyklische Diene als Ausgangssubstrat zum Einsatz kommen. Allerdings werden hierbei nur Diene mit einer maximalen Ringgröße von 8 Kohlenstoffatomen beschrieben.

Gegenstand der EP 2364965 A1 (KANEDA) ist ferner ein Verfahren zur Herstellung von Ketonen, bei dem man Olefine (z.B. Cycloocten) mit Sauerstoff in Gegenwart von Pd-Katalysatoren in amidischen Lösungsmitteln oxidiert. Als Pd-Katalysatoren kommen die Halogenide sowie deren Komplexe mit den amidischen Lösungsmitteln in Betracht, jedoch keine Pd-Ligand-Systeme.

### AUFGABE DER ERFINDUNG

Makrozyklische Diene bilden mit Pd(II)-Spezies/Verbindungen einen Chelatkomplex. Durch die räumliche Anordnung des Liganden und seine Flexibilität wird jedoch die weitere Koordination der reagierenden Komponenten behindert, wodurch deutlich die Aktivität und Selektivität der Reaktion eingeschränkt wird. Dies ist eine der Ursachen, warum solche makrozyklische Diene in den aus dem Stand der Technik bekannten Verfahren nur sehr schlecht reagieren. Zudem wird dies dadurch bestärkt, dass diese sehr unpolaren makrozyklischen Diene nur eine begrenzte Löslichkeit in polaren Lösungsmitteln aufweisen. Zufriedenstellende Ausbeuten lassen sich folglich mit den aus dem Stand der Technik bekannten Verfahren nicht erzielen.

Aufgabe der vorliegenden Erfindung war es daher, ein Verfahren bereitzustellen, das die Nachteile des Stands der Technik überwinden. Konkret war es die Aufgabe ein Verfahren bereitzustellen, welches eine einfache, material- und energiesparende und somit nachhaltige Prozessführung ermöglicht und durch welches gleichzeitig wirtschaftliche Ausbeuten erzielt werden.

### BESCHREIBUNG DER ERFINDUNG

Gegenstand ist ein Verfahren zur Herstellung von ungesättigten makrozyklischen Monoketonen umfassend die folgenden Schritte:
(a) Bereitstellung von makrozyklischen Dienen mit einer Ringgröße von mindestens 9 Kohlenstoffatomen;
(b) Inkontaktbringen der Ausgangsstoffe aus Schritt (a) mit
   (b1) einem Palladium(II)-Salz und/oder einem Palladium(II)-Komplex; und
   (b2) einem Oxidationsmittel; und
   (b3) einem Lösungsmittel;
   (b4) einem Co-Katalysator;
   (b5) einer Säure, und gegebenenfalls
   (b6) einem Liganden;
   wobei der Co-Katalysator (b4) ein zwei- oder dreiwertiges Eisensalz enthält oder daraus besteht, das von FeSO₄ verschieden ist und vorzugsweise Eisen(III)nitrat darstellt.

Überraschenderweise wurde gefunden, dass mittels des oben genannten Verfahrens ungesättigte makrozyklische Monoketone in deutlich höheren Ausbeuten hergestellt werden können, als dies bisher aus dem Stand der Technik bekannt war oder hätte erwartet werden können. Ebenfalls ist eine kommerzielle Verwendung der entstehenden Isomere möglich, was das Verfahren äußerst nachhaltig und effizient gestaltet.

Ein weiterer Vorteil der Erfindung ist es, dass die makrozyklischen Diene durch das erfindungsgemäße Verfahren derart effektiv umgesetzt werden, dass selbst, wenn die makrozyklischen Diene als Gemisch von E,E-, E,Z- und Z,Z-Isomeren vorliegen, die jeweils eine unterschiedliche Reaktivität aufweisen, diese selektiv zu einem Monoketon oxidiert werden können, ohne dass unerwünschte Mengen an Diketonen entstehen.

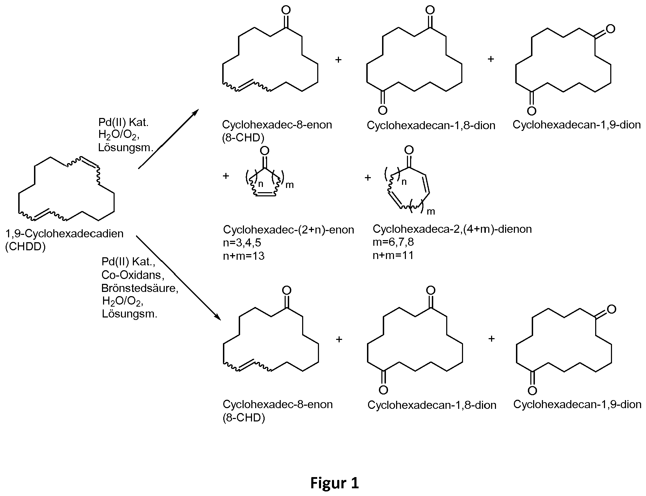

**Fig.** 1 veranschaulicht beispielhaft den Ablauf des erfindungsgemäßen Herstellungsverfahrens. Diese Abbildung stellt jedoch nur ein Beispiel dar und soll keinesfalls einschränkend sein.

Bevorzugt enthält das makrozyklische Dien (a) zwei nichtkonjugierte Doppelbindungen. In einer bevorzugten Ausführungsform der Erfindung hat das makrozyklische Dien eine Ringgröße von 9 bis 30 Kohlenstoffatomen, bevorzugt von 12 bis 18 Kohlenstoffatomen und besonders bevorzugt von 16 Kohlenstoffatomen. In eines besonders bevorzugten Ausführungsform ist das makrozyklische Dien (a) 1,9-Cyclohexadecadien (CHDD).

In einer weiteren bevorzugten Ausführungsform des erfindungsgemäßen Verfahrens ist das verwendete makrozyklische Dien (a) ein Gemisch aus verschiedenen Stereoisomeren. In einer besonders bevorzugten Ausführungsform des erfindungsgemäßen Verfahrens ist das makrozyklische Dien (a) 1,9-Cyclohexadecadien (CHDD) und liegt als Gemisch von E,E-, E,Z- und Z,Z-Isomeren vor.

Bevorzugt wird durch das erfindungsgemäße Herstellungsverfahren Cyclohexadec-8-enon (8-CHD) hergestellt. In einer besonders bevorzugten Ausführungsform der Erfindung wird als das makrozyklische Dien (a) CHDD verwendet wobei durch Anwendung der erfindungsgemäßen Herstellungsverfahrens 8-CHD erhalten wird.

In einer ersten Ausführungsform der Erfindung ist das Lösungsmittel (b3) ein polares aprotisches Lösungsmittel. Bevorzugt wird das Lösungsmittel (b3) hierbei aus der Gruppe bestehend aus N,N-disubstituierten offenkettigen und cyclische Säureamiden, wie beispielsweise Dimethylformamid, Dimethylacetamid, Diethylacetamid, Dimethylpropionamid, N-Methylpyrrolidon, aliphatische, cycloaliphatische oder aromatische Nitrile wie Acetonitril, Propionitril oder Benzonitril oder lineare und cyclische Ether, cyclische Lactone und Carbonate.

Bevorzugt werden in einer ersten Ausführungsform der Erfindung dem Lösungsmittel (b3) geringe Mengen an Wasser zugesetzt. Bevorzugt in einer Menge von 0,1 bis 25 Vol.-%, mehr bevorzugt in einer Menge von 1-20 Vol.-% und besonders bevorzugt in einer Menge von 5-15 Vol.-%. Die Volumenprozentangaben beziehen sich hierbei auf das Lösungsmittel (b3).

Weiterhin ist in einer ersten Ausführungsform der Erfindung der Ligand (b6) obligatorisch im erfindungsgemäßen Verfahren vorhanden. Bevorzugt ist der Ligand ein Bidentatligand. Der Einsatz eines Bidentatliganden ist deshalb vorteilhaft, da dieser zum einen so starke Donoreigenschaften besitzt, dass er das Palladium(II)-Salz und/oder den Palladium(II)-Komplex molekular stabilisieren und somit verhindern kann, dass Palladium(0) ausfällt. Zum anderen kann der Ligand raumgreifende Substituenten besitzen, die eine Chelatisierung von Palladium durch das Ausgangsmaterial (a) verhindern. Bevorzugt enthält der Ligand N,N-, N,O-, N,S- und/oder O,O-Donoratome. Mehr bevorzugt enthält der Ligand N,N-, N,O- und/oder O,O-Donoratome. Diese Atome können Bestandteil eines zyklischen Systems sein oder über andere geeignete verbrückende Gruppen miteinander verbunden sein.

**Fig.2** veranschaulicht Bidentatliganden, die für eine Ausführungsform des erfindungsgemäßen Verfahrens verwendet werden. Diese Abbildung ist beispielhaft und soll keine Einschränkung darstellen.

Das Palladium(II)-Salz und/oder der Palladium(II)-Komplex (b1) werden in einer ersten Ausführungsform der erfindungsgemäßen Verfahrens ausgewählt aus der Gruppe bestehend aus Palladiumbromid, Palladiumacetat, Palladiumtrifluoracetat, Palladiumbenzoat,

Tetrakis(acetonitril)palladium(II) tetrafluoroborat, Tetrakis(acetonitril)palladium (II) Bis(trifluoromethansulfonat), Palladiumnitrat und/oder Palladiumsulfat.

Weiterhin ist eine erste Ausführungsform des erfindungsgemäßen Verfahrens dadurch gekennzeichnet, dass das Palladium(II)-Salz und/oder der Palladium(II)-Komplex (b1) mit dem Bidentatligand (b6) eine Palladium-Verbindung der Formel (I) und/oder der Formel (II) bilden 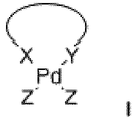 wobei X und Y jeweils unabhängig voneinander für N,N, O,N oder O,O steht; und wobei Z jeweils unabhängig voneinaner für ein Halogen, Acetat, Trimethylacetat, Trifluormethylacetat, MeCN, PhCN, NO₂, NO, Nitrat, Nitrit oder Sulfat steht; 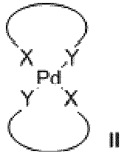 wobei X und Y jeweils unabhängig voneinander für O,O, N,N oder O,N steht.

Die so erhaltene Palladium-Verbindung in einer ersten Ausführungsform der Erfindung, liegt in einer Konzentration von 0,01 bis 25 Mol-%, bevorzugt von 1 bis 20 Mol-%, besonders bevorzugt von 1 bis 15 Mol-% und mehr bevorzugt von 1 bis 5 Mol% vor. Die Mol-% Angaben beziehen sich jeweils auf das Ausgangsmaterial (a).

Bevorzugt ist für die Palladium-Verbindung in einer ersten Ausführungsform der Erfindung ein neutraler Palladium-Katalysator.

Die Bildung der Palladium-Verbindungen nach den Formeln (I) und/oder (II) kann entweder in situ oder in einem separaten Herstellungsverfahren ex situ erfolgen. Bei einer separaten ex situ-Herstellung werden anstatt des Palladium(II)-Salzes und/oder des Palladium(II)-Komplexes (b) und der Liganden (b6) direkt die Palladium-Verbindungen mit dem Ausgangsmaterial (a) des erfindungsgemäßen Herstellungsverfahrens in Kontakt gebracht.

In einer ersten Ausführungsform der Erfindung wird als Oxidationsmittel (b2) ein sauerstoffhaltiges Gas verwendet. Bevorzugt enthält das sauerstoffhaltige Gas Sauerstoff in einer Konzentration von 1 bis 100 Vol.-%, mehr bevorzugt von 5 bis 100 Vol.-% und am meisten bevorzugt von 21 bis 100 Vol.-%.

Weiterhin wird das erfindungsgemäße Verfahren in einer ersten Ausführungsform bei erhöhten Temperaturen durchgeführt. Bevorzugt bei Temperaturen von 50°C bis 120°C, mehr bevorzugt von 60°C bis 100°C, am meisten bevorzugt von 70°C bis 90°C.

In einer zweiten Ausführungsform der Erfindung ist das Lösungsmittel (b3) ein polares aprotisches Lösungsmittel. Bevorzugt wird das Lösungsmittel (b3) hierbei aus der Gruppe bestehend aus N,N-disubstituierten offenkettigen und cyclische Säureamiden, wie beispielsweise Dimethylformamid, Dimethylacetamid, Diethylacetamid, Dimethylpropionamid, N-Methylpyrrolidon, aliphatische, cycloaliphatische oder aromatische Nitrile wie Acetonitril, Propionitril oder Benzonitril oder lineare und cyclische Ether, cyclische Lactone und Carbonate.

Im Kontext der Erfindung kann die Verwendung des Singulars wie "ein" oder "eine" oder "der, die, das" auch den Plural beinhalten, es sei denn aus dem Kontext ergibt sich eindeutig etwas anderes. Beispielsweise der Begriff "das Lösungsmittel" kann auch eine Vielzahl von Lösungsmitteln, einschließlich Mischungen davon, wie beispielsweise eine Lösungsmittelgemisch einschließen.

Bevorzugt werden in einer zweiten Ausführungsform der Erfindung dem Lösungsmittel (b3) geringe Mengen an Wasser zugesetzt. Bevorzugt in einer Menge von 0,1 bis 25 Vol.-%, mehr bevorzugt in einer Menge von 1-20 Vol.-% und besonders bevorzugt in einer Menge von 5 bis 15 Vol.-%. Die Volumenprozentangaben beziehen sich hierbei auf das Lösungsmittel (b3).

Der Co-Katalysator (b4) und die Säure (b5) sind obligatorisch im erfindungsgemäßen Verfahren vorhanden.

Als weitere Co-Katalysatoren (b4) kommen neben den eingangs genannten Eisensalzen - ausgenommen FeSO₄ - beispielsweise Chinone, Heteropolysäuren und Polyoxometallate oder Metallkomplexe, deren Zentralmetall durch Oxidation mit Sauerstoff leicht zwischen einer Oxidationsstufe II/III, II/IV, IV/V oder I/II wechseln kann, in Frage. Zentralmetalle solcher geeigneten Komplexe können beispielsweise aus der Gruppe Fe, Cu, Mn, Co, Ni, V, ausgewählt werden. Die Co-Katalysatoren werden vorzugsweise in Kombinationen eingesetzt werden, um die Elektronenübergänge während der Redoxprozesse zu unterstützen und zu erleichtern. In einer bevorzugten Ausführungsform werden die Co-Katalysatoren ausgewählt aus der Gruppe bestehend aus Benzochinonen, Napthochinonen, Anthrachinonen, Molybdatophosphorsäure, Molybdatovanadatophosphorsäuren, Wolframatomolybdatophosphorsäuren, Wolframatovanadatophosphorsäuren, Phtalocyanin-Komplexe, CuCl, CuCl₂, CuSO₄, VOSO₄. In diesen Fällen ist dann auch eine Kombination beispielsweise aus Fe(NO₃)₃ und FeSO₄, beispielsweise im Gewichtsverhältnis 1:1 möglich.

In einer zweiten Ausführungsform der Erfindung wird der Co-Katalysator (b4) bevorzugt in einer Konzentration von 1 bis 300 Mol% mehr bevorzugt von 5 bis 150 Mol% und am meisten bevorzugt von 10 bis 100 Mol% zugegeben. Die Mol%-Angaben beziehen sich jeweils auf das Ausgangsmaterial (a).

Bevorzugt ist die Säure (b5) in einer zweiten Ausführungsform der Erfindung eine Brönsted-Säure und/oder eine Lewis-Säure. Als Brönsted-Säuren sind anorganische Mineralsäuren mit schwach koordinierenden Anionen und organische Carbon-, Sulfon- und Phosphonsäuren geeignet. Besonders geeignet sind Sulfonsäuren. Zu den Lewis-Säuren zählen Verbindungen mit unvollständigem oder instabilem Elektronenoktett, wie beispielsweise B(CH₃)₃, BF₃, AlCl₃.

In einer zweiten Ausführungsform der Erfindung wird die Säure (b5) bevorzugt in einer Konzentration von 5 bis 500 Mol%, mehr bevorzugt von 15 bis 300 Mol%, noch mehr bevorzugt von 30 bis 200 Mol% und am meisten bevorzugt von 50 bis 150 Mol% zugegeben. Die Mol%-Angaben beziehen sich jeweils auf das Ausgangsmaterial (a).

In einer bevorzugten Ausführungsform der Erfindung ist die Säure eine Brönsted-Säure. Bevorzugt wird die Brönsted-Säure in einer Konzentration von 5 bis 500 Mol%, mehr bevorzugt von 15 bis 300 Mol%, noch mehr bevorzugt von 30 bis 200 Mol% und am meisten bevorzugt von 50 bis 150 Mol% zugegeben. Die Mol%-Angaben beziehen sich jeweils auf das Ausgangsmaterial (a).

Weiterhin wird das Palladium(II)-Salz und/oder der Palladium(II)-Komplex (b1) in einer zweiten Ausführungsform der Erfindung bevorzugt in einer Konzentration von 0,01 bis 20 mol%, mehr bevorzugt von 0,5 bis 10 Mol% und besonders bevorzugt von 1 bis 5 Mol-% verwendet. Die Mol%-Angaben beziehen sich jeweils auf das Ausgangsmaterial (a).

In einer zweiten Ausführungsform der Erfindung wird in einer bevorzugten Weise durch Mischen eines Palladium(II)-Salzes und/oder eines Palladium(II)-Komplexes (b1) mit einer Brönstedsäure (b5) und einem geeigneten Lösungsmittel (b3), vorzugsweise ein polar aprotisches Lösungsmittel, eine Palladium-Verbindung gebildet. Diese Palladium-Verbindung kann entweder in situ oder in einem separaten Herstellungsverfahren ex situ erfolgen. Bei einer separaten ex situ-Herstellung werden anstatt des Palladium(II)-Salzes und/oder des Palladium(II)-Komplexes (b) und der Säure (b5) die präformierten Palladium-Verbindungen mit dem Ausgangsmaterial (a) und dem Co-Katalysator (b4) sowie einem geeigneten Lösungsmittel (b3) des erfindungsgemäßen Herstellungsverfahrens in Kontakt gebracht.

In einer bevorzugten Ausführungsform ist diese Palladium-Verbindung ein kationischer Palladium-Katalysator.

In einer zweiten Ausführungsform der Erfindung wird die kationische Palladium-Verbindung bevorzugt in einer Konzentration von 0,01 Mol% bis 20 Mol%, bevorzugt in einer Konzentration von 0,5 Mol% bis 10 Mol% und besonders bevorzugt in einer Konzentration von 1 bis 5 Mol% verwendet. Die Mol%-Angaben beziehen sich hierbei jeweils auf das Ausgangsmaterial (a).

Das erfindungsgemäße Verfahren einer zweiten Ausführungsform der Erfindung wird bevorzugt bei Temperaturen zwischen 0°C und 100°C, mehr bevorzugt zwischen 0°C und 50°C, und am meisten bevorzugt zwischen 0°C und 25°C durchgeführt.

### GEWERBLICHE ANWENDBARKEIT

Gemäß der vorliegenden Erfindung können ungesättigte makrozyklische Monoketone hergestellt werden, insbesondere von makrozyklischen Dienen mit einer Ringgröße von mindestens 9 Kohlenstoffatomen; und ganz besonders bevorzugt von makrozyklischen Dienen mit einer Ringgröße von 16 Kohlenstoffatomen, die beispielsweise zu wertvollen Duft-, Aroma- oder Geschmacksstoffen weiter verarbeitet werden können.

Die durch das erfindungsgemäße Herstellungsverfahren gewonnenen ungesättigten makrozyklischen Monoketone können durch übliche Trennverfahren, beispielweise durch präparative Hochleistungsflüssigkeitschromatographie (HPLC) oder fraktionierte Destillation aufgereinigt werden.

### BEISPIELE

### Beispiele 1 und 2, Vergleichsbeispiele V1 bis V3

### DMA/MeCN/H₂O = 4/3/1; Pd(NO₃)₂ · 2 H₂O und Variation des Co-Katalysators unter reiner Sauerstoffatmosphäre)

In einem verschließbarem 4-ml-Glasreaktor mit Schraubkappe und Septum wurde ein Lösungsmittelsystem, bestehend aus *N*,*N*-Dimethylacetamid, Acetonitril und Wasser (DMA/ MeCN/H₂O *=* 4/3/1; V_{ges.} = 3 ml), 1,9-CHDD (Isomerengemisch; 44 mg; 0,2 mmol) vorgelegt. Zu dieser Lösung wurden Palladium(II)-nitrat Dihydrat (0,01 mmol; 5,0 Mol%); para-Toluolsulfonsäure Monohydrat (76 mg; 0,4 mmol) und ein Co-Katalysator (10-20 Mol%) gegeben. Der Reaktor wurde verschlossen, das Septum mit einer Einwegnadel durchstochen und in einen Autoklaven gebracht. Der Autoklav wurde mit Sauerstoff gespült und anschließend der entsprechende Sauerstoffdruck aufgepresst (1 bar). Danach wurde die Reaktionsmischung im Autoklaven bei Raumtemperatur für 18 h intensiv gerührt. Im Anschluss wurde der Autoklav entspannt und die Reaktionslösung mit Hilfe von Tetrahydrofuran auf ein konstantes Volumen aufgefüllt und homogenisiert. Die qualitative und quantitative Analyse der Reaktionsprodukte erfolgte anhand eines internen Standards (n-Hexadecan) an einem GC/MS mit FID. Die Beispiele 1 und 2 sind erfindungsgemäß, die Beispiele V1 bis V3 dienen zum Vergleich. Die Ergebnisse sind in **Tabelle 1** zusammengefasst.

**Tabelle 1**

| Variation Co-Katalysatoren | | | | | | |
|---|---|---|---|---|---|---|
| **Beispiel** | **Co-Katalysator** | **Co-Kat. [Mol%]** | **X_{1,9-CHDD} [%]** | **S_{8-CHD} [%]** | **S_{DIKETON} [%]** | **Y_{8-CHD} [%]** |
| V1 | - | - | 26 | 81 | 19 | 21 |
| V2 | Cu(NO₃)₂ · 3 H₂O | 10 | 26 | 77 | 8 | 20 |
| V3 | Benzochinon | 10 | 28 | 75 | 10 | 21 |
| 1 | Fe(NO₃)₃ · 9 H₂O | 10 | 41 | 72 | 8 | 29 |
| 2 | Fe(NO₃)₃ · 9 H₂O | 20 | 44 | 68 | 9 | 30 |

### Beispiele 3 bis 9

### DMA/MeCN/H₂O; Pd(NO₃)₂ · 2 H₂O; Fe(NO₃)₃ · 9 H₂O und Variation des Lösungsmittelverhältnisses unter reiner Sauerstoffatmosphäre

In einem verschließbarem 4-ml-Glasreaktor mit Schraubkappe und Septum wurde ein Lösungsmittelsystem, bestehend aus *N*,*N*-Dimethylacetamid, Acetonitril und Wasser (V_{ges.} = 3 ml), 1,9-CHDD (Isomerengemisch; 44 mg; 0,2 mmol) vorgelegt. Zu dieser Lösung wurden Palladium(II)-nitrat Dihydrat (0,01 mmol; 5,0 Mol%); para-Toluolsulfonsäure Monohydrat (38 mg; 0,2 mmol) und Eisen(III)-nitrat Nonahydrat (8 mg; 0,02 mmol) gegeben. Der Reaktor wurde verschlossen, das Septum mit einer Einwegnadel durchstochen und in einen Autoklaven gebracht. Der Autoklav wurde mit Sauerstoff gespült, und anschließend der entsprechende Sauerstoffdruck aufgepresst (3-5 bar). Danach wurde die Reaktionsmischung im Autoklaven bei Raumtemperatur für 18-20 h intensiv gerührt. Im Anschluss wurde der Autoklav entspannt und die Reaktionslösung mit Hilfe von Tetrahydrofuran auf ein konstantes Volumen aufgefüllt und homogenisiert. Die qualitative und quantitative Analyse der Reaktionsprodukte erfolgte anhand eines internen Standards (*n*-Hexadecan) an einem GC/MS mit FID. Die Ergebnisse sind in **Tabelle 2** zusammengefasst.

**Tabelle 2**

| Variation Lösungsmittelverhältnis | | | | | | | |
|---|---|---|---|---|---|---|---|
| **Beispiel** | **DMA/MeCN/H₂O** | **Sauerstoff [bar]** | **Zeit [h]** | **X_{1,9-CHDD} [%]** | **S_{8-CHD} [%]** | **S_{DIKETON} [%]** | **Y_{8-CHD} [%]** |
| 3 | 7/-/1 | 5 | 18 | 8 | 29 | 6 | 2 |
| 4 | 6/1/1 | 3 | 18 | 38 | 69 | 8 | 26 |
| 5 | 10/4/3 | 5 | 18 | 49 | 57 | 12 | 28 |
| 6 | 5/2/1 | 5 | 18 | 46 | 75 | 11 | 35 |
| 7 | 10/4/1 | 3 | 20 | 45 | 82 | 13 | 37 |
| 8 | 7/7/2 | 3 | 18 | 44 | 70 | 8 | 30 |
| 9 | 2/5/1 | 3 | 18 | 35 | 69 | 7 | 24 |

### Beispiele 10 bis 16

### DMA/MeCN/H₂O = 10/4/1; Pd(NO₃)₂ · 2 H₂O; Fe(NO₃)₃ · 9 H₂O und Variation der Edukt-Konzentration unter reiner Sauerstoffatmosphäre

In einem verschließbarem 4-ml-Glasreaktor mit Schraubkappe und Septum wurde ein Lösungsmittelsystem, bestehend aus N,N-Dimethylacetamid, Acetonitril und Wasser (DMA/MeCN/H₂O = 10/4/1; V_{ges.} = 3 ml), 1,9-CHDD (Isomerengemisch; 0,1-1,0 mmol) vorgelegt. Zu dieser Lösung wurden die entsprechenden Mengen an Palladium(II)-nitrat Dihydrat (5,0 Mol%); para-Toluolsulfonsäure Monohydrat (100 Mol%) und Eisen(III)-nitrat Nonahydrat (10 Mol%) gegeben. Der Reaktor wurde verschlossen, das Septum mit einer Einwegnadel durchstochen und in einen Autoklaven gebracht. Der Autoklav wurde mit Sauerstoff gespült, und anschließend der entsprechende Sauerstoffdruck aufgepresst (3-5 bar). Danach wurde die Reaktionsmischung im Autoklaven bei Raumtemperatur für 5-20 h intensiv gerührt. Im Anschluss wurde der Autoklav entspannt und die Reaktionslösung mit Hilfe von Tetrahydrofuran auf ein konstantes Volumen aufgefüllt und homogenisiert. Die qualitative und quantitative Analyse der Reaktionsprodukte erfolgte anhand eines internen Standards (n-Hexadecan) an einem GC/MS mit FID. Die Ergebnisse sind in **Tabelle 3** zusammengefasst.

**Tabelle 3**

| Variation Edukt-Konzentration | | | | | | | |
|---|---|---|---|---|---|---|---|
| **Beispiel** | **Edukt-Konzentration [mmol]** | **Sauerstoff [bar]** | **Zeit [h]** | **X_{1,9-CHDD} [%]** | **S_{8-CHD} [%]** | **S_{DIKETON} [%]** | **Y_{8-CHD} [%]** |
| 10 | 0,1 | 5 | 5 | 26 | 69 | 5 | 18 |
| 11 | 0,2 | 5 | 5 | 39 | 76 | 9 | 29 |
| 12 | 0,2 | 3 | 20 | 45 | 82 | 13 | 37 |
| 13 | 0,4 | 5 | 5 | 34 | 80 | 9 | 27 |
| 14 | 0,5 | 3 | 5 | 35 | 82 | 14 | 28 |
| 15 | 0,5 | 3 | 8 | 41 | 83 | 13 | 33 |
| 16 | 1,0 | 3 | 5 | 32 | 76 | 15 | 25 |

### Beispiele 17 bis 20

### DMA/MeCN/H₂O = 10/4/1; Pd(NO₃)₂ · 2 H₂O; Fe(NO₃)₃ · 9 H₂O und Variation der Katalysator-Konzentrationen unter reiner Sauerstoffatmosphäre

In einem verschließbarem 4-ml-Glasreaktor mit Schraubkappe und Septum wurde ein Lösungsmittelsystem, bestehend aus *N*,*N*-Dimethylacetamid, Acetonitril und Wasser (DMA/ MeCN/H₂O = 10/4/1; V_{ges.} = 3 ml), 1,9-CHDD (Isomerengemisch; 110 mg; 0,5 mmol) vorgelegt. Zu dieser Lösung wurden die entsprechenden Mengen an Palladium(II)-nitrat Dihydrat (5-10 Mol%); para-Toluolsulfonsäure Monohydrat (95 mg; 0,5 mmol) und Eisen(III)-nitrat Nonahydrat (10-20 Mol%) gegeben. Der Reaktor wurde verschlossen, das Septum mit einer Einwegnadel durchstochen und in einen Autoklaven gebracht. Der Autoklav wurde mit Sauerstoff gespült und anschließend der entsprechende Sauerstoffdruck aufgepresst (3 bar). Danach wurde die Reaktionsmischung im Autoklaven bei Raumtemperatur für 7 h intensiv gerührt. Im Anschluss wurde der Autoklav entspannt und die Reaktionslösung mit Hilfe von Tetrahydrofuran auf ein konstantes Volumen aufgefüllt und homogenisiert. Die qualitative und quantitative Analyse der Reaktionsprodukte erfolgte anhand eines internen Standards (n-Hexadecan) an einem GC/MS mit FID. Die Ergebnisse sind in **Tabelle 4** zusammengefasst.

**Tabelle 4**

| Variation Katalysator-Konzentration | | | | | | |
|---|---|---|---|---|---|---|
| **Beispiel** | **Pd(II)-Konzentration [Mol%]** | **Fe(III)-Konzentration [Mol%]** | **X_{1,9-CHDD} [%]** | **S_{8-CHD} [%]** | **S_{DIKETON} [%]** | **Y_{8-CHD} [%]** |
| 17 | 5 | 10 | 36 | 79 | 11 | 28 |
| 18 | 5 | 20 | 37 | 65 | 6 | 24 |
| 19 | 10 | 10 | 62 | 71 | 17 | 44 |
| 20 | 10 | 20 | 63 | 63 | 14 | 39 |

## Patentansprüche

1. Verfahren zur Herstellung von ungesättigten makrozyklischen Monoketonen umfassend die folgenden Schritte
(a) Bereitstellung von makrozyklischen Dienen mit einer Ringgröße von mindestens 9 Kohlenstoffatomen;
(b) Inkontaktbringen der Ausgangsstoffe aus Schritt (a) mit
(b1) einem Palladium(II)-Salz und/oder einem Palladium(II)-Komplex;
(b2) einem Oxidationsmittel;
(b3) einem Lösungsmittel;
(b4) einem Co-Katalysator;
(b5) einer Säure, und gegebenenfalls
(b6) einem Liganden;
wobei der Co-Katalysator (b4) ein zwei- oder dreiwertiges Eisensalz enthält oder daraus besteht, das von FeSO₄ verschieden ist und vorzugsweise Eisen(III)nitrat darstellt.

2. Verfahren nach Anspruch 1, **dadurch gekennzeichnet, dass** das Lösungsmittel (b3) ein polares aprotisches Lösungsmittel ist und bevorzugt ausgewählt ist aus der Gruppe bestehend aus N,N-disubstituierten offenkettigen und cyclische Säureamiden, wie beispielsweise Dimethylformamid, Dimethylacetamid, Diethylacetamid, Dimethylpropionamid, N-Methylpyrrolidon, aliphatische, cycloaliphatische oder aromatische Nitrile wie Acetonitril, Propionitril oder Benzonitril oder lineare und cyclische Ether, cyclische Carbonate und Lactone.

3. Verfahren nach den Ansprüchen 1 und/oder 2, **dadurch gekennzeichnet, dass** der Ligand (b6) obligatorisch vorhanden ist, wobei der Ligand ein Bidentatligand ist und N,N-, N,O-, N,S- oder O,O-Donoratome enthält.

4. Verfahren nach einem der Ansprüche 1 bis 3, **dadurch gekennzeichnet, dass** das Palladium(II)-Salz und/oder der Palladium(II)-Komplex (b1) ausgewählt sind aus der Gruppe bestehend aus Palladiumbromid, Palladiumacetat, Palladiumtrifluoroacetat, Palladiumbenzoat, Palladiumnitrat, Palladiumsulfat, Tetrakis(acetonitril)palladium(II) tetrafluoroborat und/oder Tetrakis(acetonitril)palladium(II) bis(trifluoromethanesulfonat).

5. Verfahren nach einem der Ansprüche 1 bis 4, **dadurch gekennzeichnet, dass** das Palladium(II)-Salz und/oder der Palladium(II)-Komplex (b1) mit dem Bidentatligand (b4) einen Palladium-Verbindung der Formel (I) und/oder der Formel (II) bilden 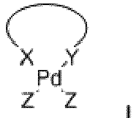 wobei X und Y jeweils unabhängig voneinander für N,N, O,N oder O,O steht; und wobei Z jeweils unabhängig voneinander für ein Halogen, Acetat, Trimethylacetat, Trifluormethylacetat, MeCN, PhCN, NO₂, NO, Nitrat, Nitrit oder Sulfat steht; 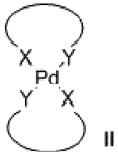 wobei X und Y jeweils unabhängig voneinander für O,O, N,N oder O,N steht

6. Verfahren nach Anspruch 5, **dadurch gekennzeichnet, dass** die Palladium-Verbindung in einer Konzentration von 0,01 bis 25 Mol%, bevorzugt von 1 bis 20 Mol%, besonders bevorzugt von 1 bis 5 Mol%, jeweils bezogen auf das Ausgangsmaterial (a), zugegeben wird.

7. Verfahren nach einem der Ansprüche 1 bis 6, **dadurch gekennzeichnet, dass** als Oxidationsmittel (b2) ein sauerstoffhaltiges Gas verwendet wird.

8. Verfahren nach Anspruch 7, **dadurch gekennzeichnet, dass** das sauerstoffhaltige Gas Sauerstoff in einer Konzentration von 1 bis 100 Vol.-%, bevorzugt von 5 bis 100 Vol.-% und am meisten bevorzugt von 10 bis 100 Vol.-% enthält.

9. Verfahren nach einem der Ansprüche 1 bis 8, **dadurch gekennzeichnet, dass** das Verfahren bei Temperaturen von 0°C und 100°C, bevorzugt zwischen 0°C und 50°C, und am meisten bevorzugt zwischen 0°C und 25°C durchgeführt wird.

10. Verfahren nach Anspruch 1 bis 9, **dadurch gekennzeichnet, dass**
(i) der Co-Katalysator (b4) mindestens eine weitere Komponente umfasst, die ausgewählt ist aus der Gruppe bestehend aus Benzochinonen, Naphthochinonen, Anthrachinonen, Molybdatophosphorsäure, Molybdatovanadatophosphorsäuren, Wolframatomolybdatophosphorsäuren, Wolframatovanadatophosphorsäuren, Phthalocyanin-Komplexe, FeSO₄, CuCl, CuCl₂, CuSO₄, VOSO₄ und
(ii) die Säure (b) eine Brönsted-Säure oder Lewis-Säure darstellt.

11. Verfahren nach Anspruch 1 bis 10, **dadurch gekennzeichnet, dass** der Co-Katalysator (b4) in einer Konzentration von 1 bis 300 Mol%, bevorzugt von 5 bis 150 Mol% und am meisten bevorzugt von 10 bis 100 Mol%, jeweils bezogen auf das Ausgangsmaterial (a), zugegeben wird.

12. Verfahren nach Anspruch 1 bis 11, **dadurch gekennzeichnet, dass** die Säure (b5) in einer Konzentration von 5 bis 500 Mol%, bevorzugt von 15 bis 300 Mol%, mehr bevorzugt von 30 bis 200 Mol% und am meisten bevorzugt von 50 bis 150 Mol%, jeweils bezogen auf das Ausgangsmaterial (a), zugegeben wird.

13. Verfahren nach Anspruch 1 bis 12, **dadurch gekennzeichnet, dass** das Palladium(II)-Salz und/oder der Palladium(II)-Komplex (b1) in einer Konzentration von 0,01 bis 20 Mol%, bevorzugt von 0,5 bis 10 Mol% und besonders bevorzugt von 1 bis 5 Mol%, jeweils bezogen auf das Ausgangsmaterial (a), zugegeben wird.

14. Verfahren nach Anspruch 1 bis 13, **dadurch gekennzeichnet, dass** das Verfahren bei Temperaturen zwischen 0°C und 100°C, bevorzugt zwischen 0°C und 50°C, und am meisten bevorzugt zwischen 0°C und 25°C durchgeführt wird.

## Claims

1. A process for the preparation of unsaturated macrocyclic monoketones comprising the following steps
(a) providing macrocyclic dienes having a ring size of at least 9 carbon atoms;
(b) contacting the starting materials from step (a) with
(b1) a palladium(II) salt and/or a palladium(II) complex;
(b2) an oxidizing agent;
(b3) a solvent;
(b4) a co-catalyst;
(b5) an acid; and optionally
(b6) a ligand;
whereby the co-catalyst (b4) comprises or consists of a di- or trivalent iron salt other than FeSO₄, preferably iron (III) nitrate.

2. The process according to claim 1, **characterized in that** the solvent (b3) is a polar aprotic solvent and is preferably selected from the group consisting of N,N-disubstituted open-chain and cyclic acid amides, such as dimethylformamide, dimethylacetamide , diethylacetamide, dimethylpropionamide, N-methylpyrrolidone, aliphatic, cycloaliphatic or aromatic nitriles such as acetonitrile, propionitrile or benzonitrile, or linear and cyclic ethers, cyclic carbonates and lactones.

3. The process as claimed in claims 1 and/or 2, **characterized in that** the ligand (b4) is ob-ligatorily present, the ligand being a bidentate ligand and containing N,N-, N,O-, N,S- or O,O-donor atoms.

4. The process according to one of claims 1 to 3, **characterized in that** the palladium(II) salt and/or the palladium(II) complex (b1) are selected from the group consisting of palladium bromide, palladium acetate, palladium trifluoroacetate, palladium adium benzoate, palladium nitrate, palladium sulfate, tetrakis(acetonitrile)palladium(II) tetrafluoroborate and/or tetrakis(acetonitrile)palladium(II) bis(trifluoromethanesulfonate).

5. The process according to any one of claims 1 to 4, **characterized in that** the palladium (II) salt and/or the palladium (II) complex (b1) with the bidentate ligand (b4) form a palladium compound of formula (I) and/or formula (II) 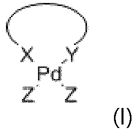 , wherein each of X and Y independently represents N,N, O,N or O,O; and wherein each Z is independently halo, acetate, trimethyl acetate, trifluoromethyl acetate, MeCN, PhCN, NO₂, NO, nitrate, nitrite or sulfate; 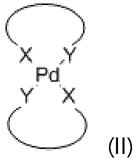 wherein X and Y are each independently of the other O, O, N, N or O, N.

6. The process according to claim 5, **characterized in that** the palladium compound is added in a concentration of from 0.01 to 25 mol-%, preferably from 1 to 20 mol-%, particularly preferably from 1 to 5 mol-%, based in each case on the starting material (a).

7. The process according to any one of claims 1 to 6, **characterized in that** an oxygen-containing gas is used as oxidant (b2).

8. The process according to claim 7, **characterized in that** the oxygen-containing gas contains oxygen in a concentration of 1 to 100% by volume, preferably of 5 to 100% by volume and most preferably of 10 to 100% by volume.

9. The process according to any one of claims 1 to 8, **characterized in that** the process is carried out at temperatures from 0°C to 100°C, preferably between 0°C and 50°C, and most preferably between 0°C and 25°C.

10. The process according to any one of claims 1 to 9, **characterized in that**
(i) the co-catalyst (b5) comprises at least one further component selected from the group consisting of benzoquinones, naphthoquinones, anthraquinones, molybdato phosphoric acid, molybdato vanadate phosphoric acids, tungstate molybdate phosphoric acids, tungstate vanadate phosphoric acids, phthalocyanine complexes, FeSO₄, CuCl, CuCl₂, CuSO₄, VOSO₄ and
(ii) the acid (b) is a Bronsted acid or Lewis acid.

11. The process according to any one of claims 1 to 10, **characterized in that** the co-catalyst (b5) is added in a concentration of from 1 to 300 mol-%, preferably from 5 to 150 mol-% and most preferably from 10 to 100 mol-%, each with respect to the starting material (a).

12. The process according to any one of claims 1 to 11, which comprises adding the acid (b6) in a concentration of from 5 to 500 mol-%, preferably from 15 to 300 mol-%, more preferably from 30 to 200 mol-% and most preferably from 50 to 150 mol-%, in each case based on the starting material (a).

13. The process according to any one of claims 1 to 12, which comprises adding the palladium(II) salt and/or the palladium(II) complex (b1) in a concentration of from 0.01 to 20 mol%, preferably from 0.5 to 10 mol% and particularly preferably from 1 to 5 mol%, in each case based on the starting material (a).

14. The process according to any one of claims 1 to 13, **characterized in that** the process is carried out at temperatures between 0°C and 100°C, preferably between 0°C and 50°C, and most preferably between 0°C and 25°C.

## Revendications

1. Procédé de préparation de monocétones macrocycliques insaturées comprenant les étapes suivantes
(a) fournir des diènes macrocycliques ayant un cycle d'au moins 9 atomes de carbone;
(b) mise en contact des matériaux de départ de l'étape (a) avec
(b1) un sel de palladium(Il) et/ou un complexe de palladium(II) ;
(b2) un agent oxydant ;
(b3) un solvant ;
(b4) un co-catalyseur ;
(b5) un acide ; et éventuellement
(b6) un ligand ;
le cocatalyseur (b4) comprend ou consiste en un sel de fer di- ou trivalent autre que le _{FeSO4}, de préférence le nitrate de fer (III).

2. Procédé selon la revendication 1, **caractérisé par le fait que** le solvant (b3) est un solvant polaire aprotique et est de préférence choisi dans le groupe constitué d'amides d'acides cycliques et à chaîne ouverte N,N-disubstitués, tels que le diméthylformamide, diméthy-lacétamide, diéthylacétamide, diméthylpropionamide, N-méthylpyrrolidone, nitriles aliphatiques, cycloaliphatiques ou aromatiques tels que l'acétonitrile, le propionitrile ou le benzonitrile, ou éthers linéaires et cycliques, carbonates cycliques et lactones.

3. Procédé selon les revendications 1 et/ou 2, **caractérisé par** la présence obligatoire du ligand (b4), le ligand étant un ligand bidentate et contenant des atomes donneurs N,N-, N,O-, N,S- ou O,O.

4. Procédé selon l'une des revendications 1 à 3, **caractérisé par le fait que** le sel de palladium(II) et/ou le complexe de palladium(II) (b1) sont choisis dans le groupe constitué par le bromure de palladium, l'acétate de palladium, le trifluoroacétate de palladium, l'acide sulfurique de palladium et l'acide sulfurique de palladium, l'acétate de palladium, le trifluoroacétate de palladium, le benzoate d'adium de palladium, le nitrate de palladium, le sulfate de palladium, le tétrafluoroborate de tétrakis(acétonitrile)palladium(II) et/ou le bis(trifluorométhanesulfonate) de tétrakis(acétonitrile)palladium(II).

5. Procédé selon l'une quelconque des revendications 1 à 4, **caractérisé par le fait que** le sel de palladium (II) et/ou le complexe de palladium (II) (b1) avec le ligand bidentate (b4) forment un composé de palladium de formule (I) et/ou de formule (II). 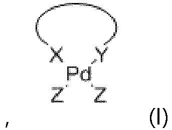 dans laquelle X et Y représentent indépendamment N,N, O,N ou O,O ; et dans laquelle Z représente indépendamment halo, acétate, acétate de triméthyle, acétate de trifluo-rométhyle, MeCN, PhCN, _{NO2}, NO, nitrate, nitrite ou sulfate ; 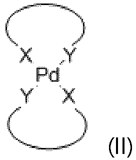 où X et Y sont indépendamment l'un de l'autre O, O, N, N ou O, N.

6. Procédé selon la revendication 5, **caractérisé par le fait que** le composé de palladium est ajouté à une concentration de 0,01 à 25 % en moles, de préférence de 1 à 20 % en moles, de préférence encore de 1 à 5 % en moles, sur la base du produit de départ (a).

7. Procédé selon l'une quelconque des revendications 1 à 6, **caractérisé par** l'utilisation d'un gaz contenant de l'oxygène comme oxydant (b2).

8. Procédé selon la revendication 7, **caractérisé par le fait que** le gaz contenant de l'oxygène contient de l'oxygène à une concentration de 1 à 100% en volume, de préférence de 5 à 100% en volume et de préférence encore de 10 à 100% en volume.

9. Procédé selon l'une quelconque des revendications 1 à 8, **caractérisé en ce que** le procédé est mis en œuvre à des températures comprises entre 0°C et 100°C, de préférence entre 0°C et 50°C, et plus préférentiellement entre 0°C et 25°C.

10. Procédé selon l'une quelconque des revendications 1 à 9, **caractérisé en ce que**
(i) le co-catalyseur (b5) comprend au moins un autre composant choisi dans le groupe constitué par les benzoquinones, les naphtoquinones, les anthraquinones, l'acide molybdato phosphorique, les acides phosphoriques molybdato vanadate, les acides phosphoriques molybdate tungstate, les acides phosphoriques vanadate tungstate, les complexes de phtalocyanine, _{FeSO4}, CuCl, CuCl₂, CuSO₄, VOSO₄ et
(ii) l'acide b) est un acide de Bronsted ou un acide de Lewis.

11. Procédé selon l'une quelconque des revendications 1 à 10, **caractérisé par le fait que** le co-catalyseur (b5) est ajouté à une concentration de 1 à 300 % en moles, de préférence de 5 à 150 % en moles et de préférence encore de 10 à 100 % en moles, chacun par rapport au produit de départ (a).

12. Procédé selon l'une quelconque des revendications 1 à 11, qui comprend l'ajout de l'acide (b6) à une concentration de 5 à 500 mol-%, de préférence de 15 à 300 mol-%, plus préférentiellement de 30 à 200 mol-% et très préférentiellement de 50 à 150 mol-%, dans chaque cas par rapport au matériau de départ (a).

13. Procédé selon l'une quelconque des revendications 1 à 12, qui comprend l'ajout du sel de palladium(Il) et/ou du complexe de palladium(II) (b1) à une concentration de 0,01 à 20 % en moles, de préférence de 0,5 à 10 % en moles et de manière particulièrement préférée de 1 à 5 % en moles, dans chaque cas sur la base du produit de départ (a).

14. Procédé selon l'une quelconque des revendications 1 à 13, **caractérisé par le fait que** le procédé est mis en œuvre à des températures comprises entre 0°C et 100°C, de préférence entre 0°C et 50°C, et plus préférentiellement entre 0°C et 25°C.
